# EUROPEAN PATENT APPLICATION

(11) **EP 3 275 458 A1**
(43) Date of publication of application: **31.01.2018**
(21) Application number: 16181521.2
(22) Date of filing: 27.07.2016
(51) Int. Cl.: A61K 38/00, A61K 31/00, A61P 7/06

(54) **COMBINATION THERAPY TO TREAT SICKLE CELL DISEASE AND RESTORE RBC FUNCTIONS**

(71) Applicant: Hartis-Pharma Sarl, 1260 Nyon (CH)
(72) Inventor: Joseph Eric, Niesor, 1260 Nyon (CH); Renée, Benghozi, 94220 Charenton le Pont (CH); François, Lamour, 68730 Blotzheim (FR)
(74) Representative: reuteler & cie SA

(57) **Abstract**

Disclosed is a new combination therapy to treat sickle cell disease (SCD) and thalassemia and their vascular acute and chronic complications by a therapy involving the combined administration of a compound improving HDL particle function and activity (i.e. increasing HDL/ ApoA1 levels), and liposoluble antioxidants xanthophylls (such as lutein, zeaxanthin, meso-zeaxanthin, astaxanthin). This method simultaneously and synergistically restores red blood cell function, normalizes red blood cell membrane cholesterol concentration and decreases oxidative stress both parameters being pathologically affected in SCD, thalassemia and diabetes secondary to low levels of plasma HDL and decreased absorption of dietary lipophilic antioxidant.

Although it is known that HDL is involved in the selective and active intestinal uptake of liposoluble vitamins and antioxidants and that HDL uniquely removes excess membrane cholesterol from red blood cells, we discovered that the combination of an HDL therapeutic intervention together with a dietary supplement (as a free or fixed combination) of liposoluble antioxidants act synergistically for treating the rheological complications of sickle cell disease as well as thalassemia and subsequently will contribute to treating and preventing microvascular damages and associated pathologies (retinopathies, kidney failure, leg ulcer, priapism, acute chest syndrome, stroke etc.). Similarly Diabetes, which includes hemorheological abnormalities associated with high red blood cell membrane cholesterol and low plasma HDL antioxidant level will benefit from such combinations. Additional compounds affecting HDL are ApoA1 inducers, HDL and ApoA1 mimetics or (mimicking compounds) ApoA1 and ApoE analogues, Lecithin Cholesterol Acyl Transferase (LCAT) activators, CETP inhibitors and modulators, Peroxisome Proliferator Activated Receptor (PPAR) agonists, ATP Binding Cassette A1 (ABCA1) inducers, Niacin and analogues, Niacin Receptor agonists. Additional lipophilic antioxidants are tocopherols and tocotrienols.

## Description

### Cross references related to application:

□ United States Patent Application Publication Pub. No.: US 2014/0023631 A1 AUERBACH et al. Pub. Date: Jan. 23, 2014
□ United States Patent Application Publication Pub. No.: US 2008/0096900 A1 Kayser et al. Pub. Date: Apr. 24, 2008
□ World Intellectual Property Organization WO2008/002591A2 Kayser et al International Publication Date : Jan. 3, 2008
□ United States Patent Application Publication Pub. No. : US 9,006,408B2 Zhou et al Pub. Date: Apr.14, 2015
□ World Intellectual Property Organization WO2015/091410A1 Chaput and Niesor al International Publication Date: Jun. 25, 2015
□ United States Patent Application Publication Pub. No. : US 7,332,514B2 Maeda et al Pub. Date: Fev.19, 2008

Background

### Sickle Cell Disease (SCD) and Thalassemia are associated with an increase in Red Blood Cell (RBC) membrane cholesterol

Sickle Cell Anemia (SCA) is the common manifestation of SCD and most frequent hemoglobinopathy in the world, affecting over 50 million people worldwide. SCA significantly impairs quality of life and shortens lifespan. While hemolytic anemia and frequent vaso-occlusive crises are the most frequent complications for patients suffering from SCA, these patients are also at very high risk for developing pulmonary hypertension, cerebral vasculopathy leading to stroke, osteonecrosis, retinopathy, priapism, leg ulcers, acute chest syndrome and glomerulopathy [1]. Sickle RBCs are very rigid and fragile. The loss of RBC deformability is considered to be the primary factor responsible for the vaso-occlusive events, severe hemolytic anemia and progressive organ damages in patients suffering from above mentioned diseases. It should also be noted that a high concentration of cholesterol in RBC decreases its capacity to transport oxygen [2, 3] thus detrimental to and triggering the sickling process. SCD and Thalassemia are characterized by low plasma HDL levels [4], high red blood cell membrane cholesterol and low blood levels of lipophilic antioxidant [5]. It is estimated that 5% of the world population is affected by these hemoglobinopathies for which very few treatments are available. Low HDL levels, high RBC membrane cholesterol, high blood viscosity [6] and low blood levels of lipophilic antioxidant, are also observed in more common pathologies of the general population such as diabetes [7] and cardiovascular diseases [8] [9] and may be at the origin of diabetes complications.

Attempts to address simultaneously the common features linking these disregulations affecting SCD, thalassemia and diabetes have not been made and vascular as well as microvascular complications of these diseases are still un-met medical needs.

### High Red Blood Cell Cholesterol is associated with low plasma HDL in SCD

Several studies reported decreased high-density lipoprotein-cholesterol (HDL-C) level in SCD [10], which may lead to an increased risk for endothelial dysfunction for patients with this disease [11]. This association could be related to the release [12] of oxidized fatty acids during lipolysis, leading to endothelial cell inflammation [11].

Although sharing common mechanisms with atherosclerosis (oxidative stress, inflammation and vascular adhesion), SCA vasculopathy clearly differs in that cholesterol accumulation in arterial wall and atheromas have not been reported [13]. Intriguingly, while plasma total lipids and cholesterols levels are usually lower in SCA than in healthy individuals, the level of total lipids and cholesterol is higher in the RBC membrane of SCA patients compared to controls [14], which could be related to the decreased plasma Lecithin Cholesterol Acyl Transferase (LCAT) level found in SCA patients, and more particularly during vaso-occlusive crises [15]. Increased RBC membrane cholesterol content even in the absence of sickle cell disease patients has been shown to strongly affect the deformability and visco-elasticity properties of the cells [16] [17] and is thus directly affecting these properties.

Indeed, the increased cholesterol content in the RBC membranes of SCA patients could participate to the loss of their deformability. Consequently, in SCA patients, one could suggest that the loss of RBC deformability caused by the sickling process and the accumulation of cholesterol into the membranes could negatively interfere with the release of ATP under deformation. This phenomenon would secondarily impact the vascular function which is known to be decreased in SCA patients [18] [19].

Increased RBC adhesiveness in SCA also contributes to the initiation of vaso-occlusive crises [20] [21]. Epinephrine (a major beta adrenergic receptor agonist) has been shown to promote the adhesion of sickle RBC to laminin [20] through its effects on Lu/BCAM phosphorylation by the adenylate cyclase/cAMP/protein Kinase A pathway [22]. Eyler et al [23] demonstrated that polymorphisms of the β2-adrenergic receptor (ADRB2) and adenylate cyclase 6 (ADCY6) genes are affecting RBC adhesion to laminin. The authors, and others [24], thus proposed that ADRB2 and adenylyl cyclases polymorphisms could influence SCA severity through the RBC adhesion mechanism. Since membrane cholesterol has been found to enhance the beta-2-adrenergic signaling pathway [25], it has been postulated, that the high membrane cholesterol level in sickle RBCs might affect the beta-2-adrenergic response to epinephrine, leading to an increased adhesiveness to endothelial cells.

Patients with Sickle Cell Disease and Thalassemia have repeatedly been observed to display low HDL-C and low LDL-C plasma levels but increase oxidized LDL and plasma levels for the markers of oxidation [38] [39]. The role of HDL in removing cholesterol from peripheral tissues such as macrophages is well established, in contrast to its role in removing excess cholesterol from RBC which has been and is still poorly investigated with very restricted therapeutic perspectives. As early as 1976, RBC from SCD patients were observed to be significantly enriched in cholesterol as compared to heathy control subjects. The difference was clearly established even considering the small sample size evaluated in the studies (patient numbers as low as 10 to 30) [40]. Schwartz et al [41] evidenced that the RBC cholesterol pool is a large pool exchanging rapidly with plasma lipoproteins and the authors suggested a central role of LCAT in this process. Importantly, Suda et al [42] confirmed the critical role of LCAT in the maintenance of RBC cholesterol and its association with anemia and red cell fragility frequently observed in LCAT deficient patients.

It was also reported that ApoA1 level (the major protein component of HDL) is decreased in SCA [26] and that patients with the lowest Apo-A1 levels had blunted flow-mediated dilation in response to acetylcholine and had higher prevalence of pulmonary hypertension [11].

### Plasma levels of lipophilic antioxidant is low in Sickle Disease and Thalassemia

Sickle cell disease, thalassemia, and glucose-6-phosphate-dehydrogenase deficiency are all hereditary disorders with higher potential for oxidative damage due to chronic redox imbalance in red cells that often results in clinical manifestation of mild to severe hemolysis in patients with these disorders. Hamdy et al [5] found a decreased antioxidant level in both SCD and thalassemia and considered accelerated oxidative damage as one of the hallmarks in both SCD and thalassemia major. More importantly, Tangney et al [27] observed that although dietary analyses suggest that dietary intakes of SCA individuals exceeded the recommended daily allowances (RDA) of all macro- and micronutrients measured (specifically, beta carotene, alpha carotene, and cryptoxanthin), all serum values for carotenoids examined, specifically, beta carotene, alpha carotene, and cryptoxanthin were markedly depressed when compared to those of healthy controls. These results suggest that in individuals with SCA, several micronutrients vital for maintaining reducing capacity are present in lower quantities in plasma/serum.

In addition Natta et al [28] measured a decrease of major carotenoids, beta carotene, cryptoxanthin, lycopene lutein, as well as alpha-tocopherol and retinol in plasma of SCA patients.

Vitamin E levels in plasma have been consistently found to be decreased in patients suffering from thalassemia. For example Zannos-Mariolea et al [29] reported that serum vitamin E levels was below normal (<0.5 mg/100 ml) in 46% of the 56 children with major beta-thalassaemia (P <0.001) evaluated in their study.

The low level of plasma lipophilic antioxidant in SCD and thalassemia may enhance the pathological manifestations of increased oxidative stress well established in these diseases. It is important to note that attempts to provide vitamin E as dietary supplement alone have not been successful to ameliorate SCD pathologies [30].

From a review of therapeutic approaches to treat the oxidative status of SCD, vitamin E and other lipophilic antioxidant have not been considered as of therapeutic potential treatment of SCD [31]. In addition from the most recent review [32] of current preclinical and clinical studies investigating potential treatments of SCD none of the components of the therapeutic approach of the current application (HDL, liposoluble vitamins) or their combination are considered.

Thus the discovery of the applicant is unique and provides a novel therapeutic approach to treat and possibly prevent efficiently the key pathological features of this disease with unmet medical needs.

### HDL plays an important role in the intestinal uptake and transport of lipophilic antioxidant

HDL has been recognized to have antioxidant properties and to protect LDL from oxidation in vivo [28] and in vitro [29]. This potential atheroprotective activity has been attributed to enzymes bound to HDL, such as paraoxonase 1 (PON1) and matrix metallopeptidase 2 (MMP2) or antioxidants such as vitamin E, lutein and zeaxanthin. Hirowatari et al [30] showed that the relative amount of the vitamin E isomers alpha plus gamma tocopherol in HDL is 5.82 nmol/mol cholesterol whereas it is only 3.55 in LDL. Similarly, lutein and zeaxanthin are preferentially transported by HDL: the ratio of lutein and zeaxanthin in HDL:LDL is 2.49 and 1.95, respectively. For alpha and beta-carotene this ratio is 0.45 and 0.31, respectively [31]. As expected from their relative lipophilicity, the bulk of tocopherol and carotene are transported by LDL (45% and 76%, respectively), whereas the oxygenated antioxidant carotenoids (lutein, zeaxanthin, astaxanthin and beta-cryptoxanthin) are enriched in HDL [32]. Thus, HDL preferentially carries a number of lipophilic antioxidants (vitamin E, lutein and zeaxanthin) that are very likely to contribute to its antioxidative properties. This has been demonstrated in vitro by the unique property of HDL enriched with these antioxidants to protect LDL from oxidation [33]. Since vitamin E, lutein and zeaxanthin are exclusively obtained from the diet and are transported by plasma lipoproteins, the pathways involved in their intestinal absorption are of critical importance. Until recently, these antioxidants were thought to be absorbed exclusively via a chylomicron-mediated pathway [34] and were assumed to be redistributed among lipoproteins during secretion of VLDL by the liver, or via an exchange proteins such as CETP or phospholipid transfer protein (PLTP) [35]. Using Caco-2 cells, Nicod et al [36] demonstrated that Vitamin E is also absorbed and directly delivered to ApoA1 via ABCA1, however not directly to mature HDL. In vivo studies using ABCA1 KO mice have shown that plasma gamma-tocopherol levels were four-fold lower in ABCA1 KO mice than in wild-type mice, whereas retinyl ester plasma levels did not differ between animal strains [34]. The mechanism appeared to be enantioselective, since alpha-tocopherol and gamma-tocopherol were preferentially absorbed compared with delta-tocopherol [36]. Thus, although, the amount of vitamin E delivered directly to plasma HDL may be less compared to the chylomicron pathway; it may be more selective and may allow delivery to tissues that do not express the ApoB receptor, but express instead the HDL receptor SRB1. Using hamsters fed with a lutein and zeaxanthin supplemented diet we demonstrated that treatment with dalcetrapib (a CETP modulator), which increases the remodeling of HDL to produce free ApoA1, led to increased lutein and zeaxanthin uptake [37].

### Definitions

### HDL, HDL enhancers, HDL peptide mimetics, injectable HDL analogues

### HDL

Plasma HDL is formed through the interaction between ApoA1 and ABCA1 the later effluxing numerous lipophilic molecules (such as cholesterol, phospholipids, plant sterols, vitamin E, lutein and zeaxanthin to ApoA1 thus forming a nascent HDL particle (discoidal pre-beta1-HDL particle). LCAT activity will further increase the capacity of HDL to take-up and transport more of the above lipophilic molecules.

This cargo will be delivered to the HDL receptor SRB-1 expressing cells and/or exchanged with several tissues including the circulating RBC which are in vicinity of the plasma HDL pool.

ApoA1 displays the unique capacity of removing cholesterol from loaded cell membranes through a membrane transporter action (ABCA1 and ABCG1) or by diffusion following the concentration gradient. The phenomenon also called reverse cholesterol transport has been very well documented using cholesterol labelled-macrophages. It is to be noted that the large plasma pool of RBC cholesterol has been shown to play an important role in this process [33].

Therapeutic approaches targeting HDL have been reviewed recently by Uehara et al [34] and all of them can be considered as potential component of the combination proposed herein. In particular LCAT activators, CETP inhibitors and modulators, ApoA1 mimetics, full-length ApoA1, ApoA1-mimetic peptides such as D4F, LF4, FAMP and analogues, reconstituted HDL, including ApoA1-phospholipid complexes, ApoA1 Milano, ApoE phospholipid complexes are considered.

### HDL enhancers

Several approaches can be used to increase the plasma level of HDL/ApoA1.

Compounds enhancing ApoA1 production or plasma accumulation.

Numerous small molecules of hormone receptor activators have been shown to increase ApoA1 production. More specifically, enhancing HDL formation has been achieved by increasing ApoA1 production by tissues such as liver and intestine with PPAR alpha agonists (fibrates), Thyroid Receptor agonists and Niacin and analogues. Plasma HDL can be raised by increasing ABCA1 activity through activation of the Liver X Receptor (LXR) a direct regulator of ABCA1 gene expression.

LCAT activity is necessary to the formation of large and mature HDL particles. Human recombinant LCAT protein can be infused into animal and human to increase plasma HDL lipids and apolipoproteins [35, 36].

Similarly small molecules enhancing LCAT activity and modified LCAT protein with enhanced enzymatic activity are under development, they indeed increase plasma HDL [37]

### HDL peptide mimetics

The mature sequence of human ApoA1 has a length of 243 amino acid residues and is encoded by exons 3 and 4 of the apolipoprotein gene in chromosome 11. The common lipid-associating motif in ApoA1 is the amphipathic α helix. ApoA1 contains multiple repeats of 22 amino acids (22-mer), each of which is a tandem array of two 11-mers. A large number of peptides mimicking these repeats have been synthetized and display some of the properties of ApoA1 and especially with regard to their ability to remove cell membrane cholesterol. For instance L-4F, an ApoaA1 mimetic, dramatically improves vasodilation in hypercholesterolemia and Sickle Cell Disease.

ApoA1 mimetics, full-length ApoA1, ApoA1-mimetic peptides such as D4F, LF4, FAMP and analogues are considered. FAMP differs from other ApoA1 mimetics because it is designed to specifically interact with human ABCA1 without engaging the nonspecific, passive efflux pathway. Therefore, it functions similarly to human ApoA1. Furthermore, FAMP markedly increases pre-β HDL particles (nascent HDL particles) as well as overall cholesterol efflux from peripheral tissues.

### HDL analogues

HDL analogues are defined as any particle containing at least one HDL particle associated apolipoprotein (ApoA1, ApoA2, ApoE, ApoC1, ApoC3, ApoL, ApoM, ApoAIV etc.) alone or prepared as a lipoprotein complex. Reconstituted HDL, including ApoA1-phospholipid complexes, ApoA1 Milano, ApoA1 Paris, ApoE phospholipid complexes are more specifically considered.

### Liposoluble vitamins and antioxidants

A large number of lipophilic antioxidants are from dietary origin: tocopherols and carotenoids and are thus uniquely absorbed through the intestine and selectively transported by plasma lipoproteins chylomicrons, VLDL, LDL and HDL. Because of the apolipoprotein composition and abundance of each of these apolipoproteins in each class of lipoprotein in the plasma of individual subject the uptake, transports and delivery of these lipophilic antioxidants to specific cell type and tissue will be different.

It is thus of ultimate importance to match the level of individual lipoprotein with the one of the selected liposoluble antioxidant and the targeted cell type or tissue to be treated.

### Carotenoids

Carotenoids are one of the most widespread groups of pigments with more than 600 identified in nature. They cannot be synthetized by animal and human, and few of them are absorbed from the diet and found circulating in human plasma. In plants, cyclization of lycopene either leads to the formation of beta-carotene and its derivative xanthophylls, beta-cryptoxanthin, zeaxanthin, and violaxanthin or alpha-carotene and lutein. Carotenes are characterized by cyclization at one or both ends whereas xanthophylls are formed by the introduction of oxygen. Beside provitamin A activity, carotenoids are important as antioxidants and protective agents against various diseases.

Because of i) their potent antioxidant properties and selective uptake and transport by HDL, ii) their accumulation in important human tissues (retina, brain) and iii) their low levels in SCD, the xanthophylls, lutein, zeaxanthin, beta-cryptoxanthin, astaxanthin, and violaxanthin are the preferred supplement to be added to HDL therapeutic intervention.

### Tocopherols and Tocotrienols

There are eight naturally occurring vitamin E isoforms, alpha-, beta-, gamma- and delta-tocopherol and alpha-, beta-, gamma- and delta-tocotrienol. They are all potent liposoluble antioxidants, capable of neutralizing free radicals directly by donating hydrogen from its chromanol ring. Alpha-Tocopherol is regarded as the dominant form in vitamin E as the alpha-tocopherol transfer protein in the liver binds mainly alpha-tocopherol, thus preventing its degradation.

Combination therapy to treat the acute and chronic manifestations of SCD and Thalassemia

### HDL enhancer, HDL peptide mimetics and HDL analogues injectable and oral treatments of the acute manifestations of SCD and Thalassemia

Example 1: human recombinant LCAT rhLCAT (ACP-501) is infused intravenously over 1 hour on 3 occasions in a dose optimization phase (0.3, 3.0, and 9.0mg/kg), then 3.0 or 9.0mg/kg every 1 to 2 weeks for 7months in a maintenance phase, with the daily oral administration of 5, 10, 20mg lutein-zeaxanthin such as Floraglo®.

Example 2. ApoA1 mimetics, full-length ApoA1, ApoA1-mimetic peptides adequately formulated are administered subcutaneously, intravenously or orally at therapeutic efficacious doses with the simultaneous daily oral administration of 5 or 10 or 20mg lutein-zeaxanthin such as Floraglo.

Example 3. ApoA1, ApoA2, ApoE, ApoC1, ApoC3, ApoL, ApoM, ApoAIV protein or peptide adequately formulated are administered subcutaneously, intravenously or orally at therapeutic efficacious doses with the simultaneous daily oral administration of 5 or 10 or 20mg lutein-zeaxanthin such as Floraglo.

### Compounds administered orally and affecting HDL level and activity in combination with lipophilic antioxidant for the chronic treatment of SCD and thalassemia

Example 4. Patients are treated daily with a therapeutically active oral doses of micronized fenofibrate (for example 150mg per day) or Niacin (for example 1000mg per day) with the simultaneous daily oral administration of 5 or 10 or 20mg lutein-zeaxanthin such as Floraglo.

Example 5: Patients are treated daily with a therapeutically active oral doses of a LXR agonist with the simultaneous daily oral administration of 5 or 10 or 20mg lutein-zeaxanthin such as Floraglo.

Example 6: Oral CETP inhibitors and more specifically oral CETP modulators lead to an increase plasma levels of ApoA1 and HDL cholesterol.

Patients are treated daily with a therapeutically active oral doses of a CETP modulator for example 150, 300, 450 or 600mg of dalcetrapib with the simultaneous daily oral administration of 5 or 10 or 20mg lutein-zeaxanthin such as Floraglo.

### Summary of the invention

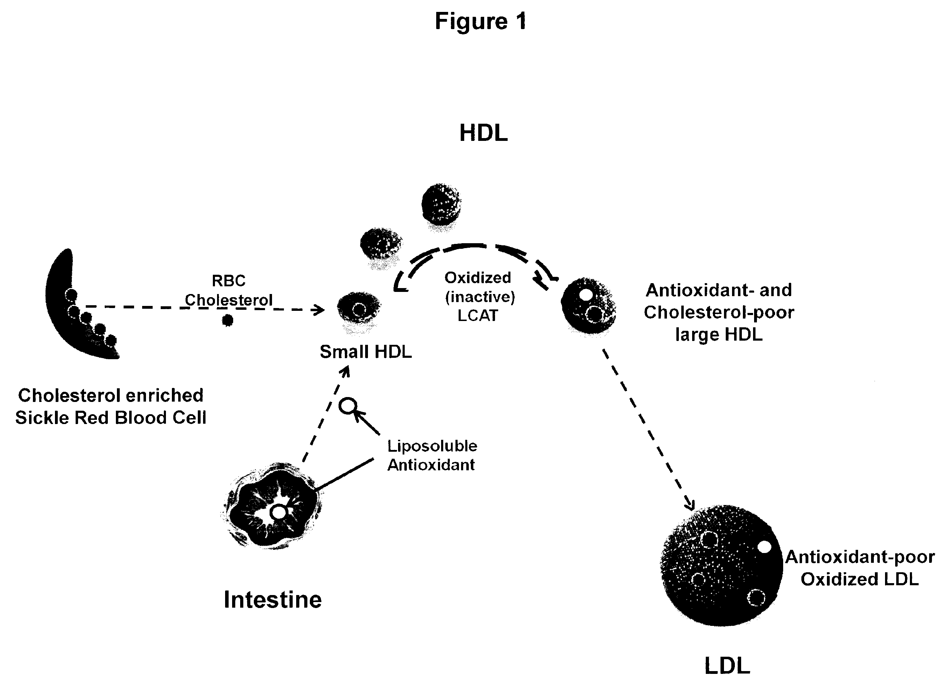

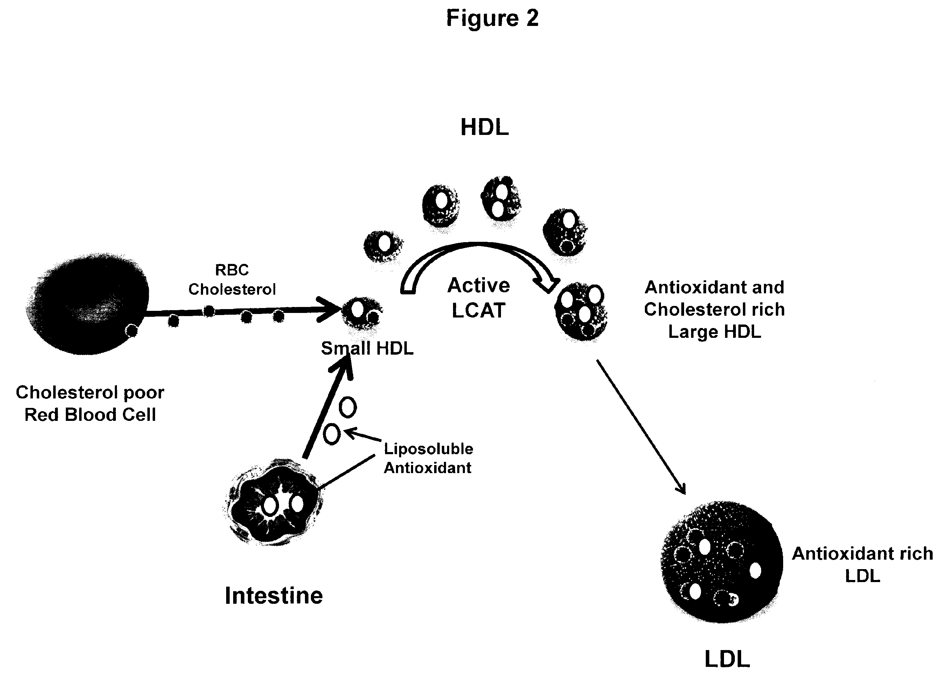

Disclosed is a new combination therapy to treat the acute and chronic complications associated with red blood cell dysfunction, increased red blood cell cholesterol (sickle cell disease, thalassemia, diabetes) and decreased plasma levels of lipophilic antioxidant by a therapy involving the combined administration of a compound improving HDL particle function and activity (i.e. increasing HDU ApoA1 levels), with the simultaneous administration of liposoluble antioxidants (xanthophylls and tocopherols).
**Claims**
1. A combination therapy to treat and prevent microvascular complications of sickle cell disease and thalassemia and restores red blood functions by administering both a compound increasing HDL activity and liposoluble antioxidants
2. A combination therapy according to claim one where HDL activity is increased by administering a recombinant Lecithin Cholesterol Acyl Transferase (LCAT) protein (i.e. ACP-501) or LCAT peptidic fragments
2. A combination therapy according to claim one where HDL activity is increased by administering apolipoprotein A1 (ApoA1), ApoA1 analogues or ApoA1 mimetic peptides such as L4F, D4F, FAMP
3. A combination therapy to treat sickle cell disease and thalassemia according to claim one where the compound mimicking ApoA1 activity is ApoE, ApoA2, ApoC1, ApoC3, ApoL, ApoM, ApoAIV or any derived or mimicking peptide
4. A combination therapy according to claim one where HDL activity is increased by administering a natural or synthetic HDL particle analogues (ie ApoA1/phospholipid mixture) such as CSL-111, CSL-112 or CER-001
5. A combination therapy according to claim one where HDL activity is increased by administering a natural or synthetic ApoA1/phospholipid mixture containing a apolipoprotein A1 variant: such as ApoA1 Milano, ApoA1 Paris etc...
6. A combination therapy according to claim one where HDL activity is increased by administering a Peroxisome Proliferator-Activated Receptor alpha or gamma such as fenofibrate or pioglitazone
7. A combination therapy according to claim one where HDL activity is increased by enhancing ATP binding cassette A1 (ABCA1) expression by for example by administering a Liver X Receptor (LXR) activator
8. A combination therapy according to claim one where HDL activity is increased by administering Niacin and analogues
9. A combination therapy according to claim one where HDL activity is increased by administering a Bromodomain and Extra-Terminal (BET) protein inhibitor such as RVX-208
10. A combination therapy according to claim one where HDL activity is increased by administering Cholesteryl Ester Transfer Protein (CETP) modulator such as dalcetrapib
11. A combination therapy according to claim one where HDL activity is increased by administering a Cholesteryl Ester Transfer Protein (CETP) inhibitor such as anacetrapib
12. A combination therapy according to claim one where the lipophilic antioxidant is a xanthophylls such as lutein, zeaxanthin, meso-zeaxanthin, astaxanthin, beta-cryptoxanthin etc...
13. A combination therapy according to claim one where the lipophilic antioxidant is a tocopherol isomer or derivative: alpha-, beta-, gamma- and delta-tocopherol
14. A combination therapy according to claim one where the lipophilic antioxidant is a tocotrienol isomer or derivative: alpha-, beta-, gamma- and delta-tocotrienol
15. A combination therapy according to claim 1 to treat microvascular complications of diabetes due to pathological red blood cell functions

### References

1. Kato, G.J., M.T. Gladwin, and M.H. Steinberg, Deconstructing sickle cell disease: reappraisal of the role of hemolysis in the development of clinical subphenotypes. Blood Rev, 2007. 21(1): p. 37-47.
2. Buchwald, H., et al., Plasma cholesterol: an influencing factor in red blood cell oxygen release and cellular oxygen availability. J Am Coll Surg, 2000. 191(5): p. 490-7.
3. Buchwald, H., et al., Effect of plasma cholesterol on red blood cell oxygen transport. Clin Exp Pharmacol Physiol, 2000. 27(12): p. 951-5.
4. Ashar, S., et al., Serum fasting lipid profile in children and adolescents with beta-thalassaemia major in southern Pakistan. Malays J Pathol, 2015. 37(3): p. 233-8.
5. Hamdy, M.M., et al., Selenium and Vitamin E as antioxidants in chronic hemolytic anemia: Are they deficient? A case-control study in a group of Egyptian children. J Adv Res, 2015. 6(6): p. 1071-7.
6. Stamos, T.D. and R.S. Rosenson, Low high density lipoprotein levels are associated with an elevated blood viscosity. Atherosclerosis, 1999. 146(1): p. 161-5.
7. Barnes, H.J., Blood rheology in diabetes mellitus. Acta Med Port, 1986. 7(5-6): p. S36-9.
8. Namazi, G., et al., Association of the total cholesterol content of erythrocyte membranes with the severity of disease in stable coronary artery disease. Cholesterol, 2014. 2014: p. 821686.
9. Simon, D.I. and R.L. Silverstein, Atherothrombosis: Seeing Red?Circulation, 2015. 132(20): p. 1860-2.
10. Seixas, M.O., et al., Levels of high-density lipoprotein cholesterol (HDL-C) among children with steady-state sickle cell disease. Lipids Health Dis, 2010. 9: p. 91.
11. Yuditskaya, S., et al., Proteomic identification of altered apolipoprotein patterns in pulmonary hypertension and vasculopathy of sickle cell disease. Blood, 2009. 113(5): p. 1122-1128.
12. Sandor, B., et al., Effects of Poloxamer 188 on red blood cell membrane properties in sickle cell anaemia. Br J Haematol, 2016. 173(1): p. 145-9.
13. Zorca, S., et al., Lipid levels in sickle-cell disease associated with haemolytic severity, vascular dysfunction and pulmonary hypertension. Br J Haematol, 2010. 149(3): p. 436-45.
14. Westerman, M.P., L.E. Pierce, and W.N. Jensen, Erythrocyte and Plasma Lipids in Sickle Cell Anemia. Blood, 1964. 23: p. 200-5.
15. Homan, R., et al., A fluorescence method to detect and quantitate sterol esterification by lecithin:cholesterol acyltransferase. Anal Biochem, 2013. 441(1): p. 80-6.
16. Chabanel, A., et al., Influence of cholesterol content on red cell membrane viscoelasticity and fluidity. Biophys J, 1983. 44(2): p. 171-6.
17. Meleard, P., et al., Bending elasticities of model membranes: influences of temperature and sterol content. Biophys J, 1997. 72(6): p. 2616-29.
18. Kato, G.J., O.C. Onyekwere, and M.T. Gladwin, Pulmonary hypertension in sickle cell disease: relevance to children. Pediatr Hematol Oncol, 2007. 24(3): p. 159-70.
19. Charlot, K., et al., Changes in autonomic nervous activity during vaso-occlusive crisis in patients with sickle cell anaemia. Br J Haematol, 2016.
20. Colin, Y., et al., Red cell and endothelial Lu/BCAM beyond sickle cell disease. Transfus Clin Biol, 2008. 15(6): p. 402-5.
21. El Nemer, W., et al., Role of Lu/BCAM in abnormal adhesion of sickle red blood cells to vascular endothelium. Transfus Clin Biol, 2008. 15(1-2): p. 29-33.
22. Zennadi, R., et al., Epinephrine-induced activation of L W-mediated sickle cell adhesion and vaso-occlusion in vivo. Blood, 2007. 110(7): p. 2708-2717.
23. Eyler, C.E., et al., beta(2)-Adrenergic receptor and adenylate cyclase gene polymorphisms affect sickle red cell adhesion. Br J Haematol, 2008. 141(1): p. 105-8.
24. Niesor, E.J., et al., Adenylyl Cyclase 9 Polymorphisms Reveal Potential Link to HDL Function and Cardiovascular Events in Multiple Pathologies: Potential Implications in Sickle Cell Disease. Cardiovasc Drugs Ther, 2015. 29(6): p. 563-572.
25. Pontier, S.M., et al., Cholesterol-dependent separation of the beta2-adrenergic receptor from its partners determines signaling efficacy: insight into nanoscale organization of signal transduction. J. Biol. Chem, 2008. 283(36): p. 24659-24672.
26. Sasaki, J., M.R. Waterman, and G.L. Cottam, Decreased apolipoprotein A-I and B content in plasma of individuals with sickle cell anemia. Clin Chem, 1986. 32(1 Pt 1): p. 226-7.
27. Tangney, C.C., et al., Selected indices of micronutrient status in adult patients with sickle cell anemia (SCA). Am J Hematol, 1989. 32(3): p. 161-6.
28. Natta, C., et al., Low serum levels of carotenoids in sickle cell anemia. Eur J Haematol, 1988. 41(2): p. 131-5.
29. Zannos-Mariolea, L., et al., Serum vitamin E levels with beta-thalassaemia major: preliminary report. Br J Haematol, 1974. 26(2): p. 193-9.
30. Muskiet, F.A., et al., Supplementation of patients with homozygous sickle cell disease with zinc, alpha-tocopherol, vitamin C, soybean oil, and fish oil. Am J Clin Nutr, 1991. 54(4): p. 736-44.
31. Vichinsky, E., Emerging 'A' therapies in hemoglobinopathies: agonists, antagonists, antioxidants, and arginine. Hematology Am Soc Hematol Educ Program, 2012. 2012: p. 271-5.
32. Archer, N., F. Galacteros, and C. Brugnara, 2015 Clinical trials update in sickle cell anemia. Am J Hematol, 2015. 90(10): p. 934-50.
33. Hung, K.T., et al., Red blood cells play a role in reverse cholesterol transport. Arterioscler Thromb Vasc Biol, 2012. 32(6): p. 1460-5.
34. Uehara, Y., G. Chiesa, and K. Saku, High-Density Lipoprotein-Targeted Therapy and Apolipoprotein A-I Mimetic Peptides. Circ J, 2015. 79(12): p. 2523-8.
35. Shamburek, R.D., et al., Familial lecithin:cholesterol acyltransferase deficiency: First-in-human treatment with enzyme replacement. J Clin Lipidol, 2016. 10(2): p. 356-67.
36. Shamburek, R.D., et al., Safety and Tolerability of ACP-501, a Recombinant Human Lecithin:Cholesterol Acyltransferase, in a Phase 1 Single-Dose Escalation Study. Circ Res, 2016. 118(1): p. 73-82.
37. Gunawardane, R.N., et al., Agonistic Human Antibodies Binding to Lecithin-Cholesterol Acyltransferase Modulate High Density Lipoprotein Metabolism. J Biol Chem, 2016. 291(6): p. 2799-811.

## Claims

**1.** A combination therapy to treat and prevent microvascular complications of sickle cell disease and thalassemia and restores red blood functions by administering both a compound increasing HDL activity and liposoluble antioxidants

**2.** A combination therapy according to claim one where HDL activity is increased by administering a recombinant Lecithin Cholesterol Acyl Transferase (LCAT) protein (i.e. ACP-501) or LCAT peptidic fragments

**2.** A combination therapy according to claim one where HDL activity is increased by administering apolipoprotein A1 (ApoA1), ApoA1 analogues or ApoA1 mimetic peptides such as L4F, D4F, FAMP

**3.** A combination therapy to treat sickle cell disease and thalassemia according to claim one where the compound mimicking ApoA1 activity is ApoE, ApoA2, ApoC1, ApoC3, ApoL, ApoM, ApoAIV or any derived or mimicking peptide

**4.** A combination therapy according to claim one where HDL activity is increased by administering a natural or synthetic HDL particle analogues (ie ApoA1/phospholipid mixture) such as CSL-111, CSL-112 or CER-001

**5.** A combination therapy according to claim one where HDL activity is increased by administering a natural or synthetic ApoA1/phospholipid mixture containing a apolipoprotein A1 variant: such as ApoA1 Milano, ApoA1 Paris etc...

**6.** A combination therapy according to claim one where HDL activity is increased by administering a Peroxisome Proliferator-Activated Receptor alpha or gamma such as fenofibrate or pioglitazone

**7.** A combination therapy according to claim one where HDL activity is increased by enhancing ATP binding cassette A1 (ABCA1) expression by for example by administering a Liver X Receptor (LXR) activator

**8.** A combination therapy according to claim one where HDL activity is increased by administering Niacin and analogues

**9.** A combination therapy according to claim one where HDL activity is increased by administering a Bromodomain and Extra-Terminal (BET) protein inhibitor such as RVX-208

**10.** A combination therapy according to claim one where HDL activity is increased by administering Cholesteryl Ester Transfer Protein (CETP) modulator such as dalcetrapib

**11.** A combination therapy according to claim one where HDL activity is increased by administering a Cholesteryl Ester Transfer Protein (CETP) inhibitor such as anacetrapib

**12.** A combination therapy according to claim one where the lipophilic antioxidant is a xanthophylls such as lutein, zeaxanthin, meso-zeaxanthin, astaxanthin, beta-cryptoxanthin etc...

**13.** A combination therapy according to claim one where the lipophilic antioxidant is a tocopherol isomer or derivative: alpha-, beta-, gamma- and delta-tocopherol

**14.** A combination therapy according to claim one where the lipophilic antioxidant is a tocotrienol isomer or derivative: alpha-, beta-, gamma- and delta-tocotrienol

**15.** A combination therapy according to claim 1 to treat microvascular complications of diabetes due to pathological red blood cell functions
